# EUROPEAN PATENT SPECIFICATION

(11) **EP 1 545 388 B1**
(45) Date of publication and mention of the grant of the patent: **05.08.2009**
(21) Application number: 03774630.2
(22) Date of filing: 02.10.2003
(51) Int. Cl.: A61F 2/01, A61M 25/06, A61M 25/00

(54) **EXPANDABLE RETRIEVAL DEVICE**
EXPANDIERBARE RÜCKHOLVORRICHTUNG
DISPOSITIF DE RECUPERATION EXTENSIBLE

(30) Priority: 02.10.2002 US 415396 P
(43) Date of publication of application: 29.06.2005
(73) Proprietor: Boston Scientific Limited, St. Michael, Barbados, West Indies (BB)
(72) Inventor: SALAHIEH, Amr, Saratoga, CA 95070 (US); SHULTZ, Michael, Santa Cruz, CA 95062 (US); LADUCA, Paul, Buffalo, NY 14216 (US); BROOME, Thomas, E., Shakopee, MN 55379 (US)
(74) Representative: Vossius & Partner
(86) International application number: PCT/US2003/031762
(87) International publication number: WO 2004/030577

(56) References cited:
- GB-A- 2 020 557
- US-A- 5 584 821
- US-B1- 6 245 089

## Description

### Field of the Invention

The present invention relates generally to the field of intravascular devices. More specifically, the present invention pertains to retrieval devices for embolic protection filters. See for example US 6 245 089.

### Background of the Invention

Intravascular devices such as an embolic protection filters are typically placed in a vessel such as an artery or vein to filter emboli contained in the blood stream. Examples of procedures employing such filters include angioplasty, atherectomy, thrombectomy, and stenting. These procedures generally involve transluminally inserting and delivering within the artery or vein an elongated wire and filter to a location distal a lesion. Once placed, a therapeutic device such as an angioplasty catheter is advanced along the wire to the site of the lesion to perform a therapeutic procedure (*e*.*g*. percutaneous transluminal coronary angioplasty). A stent can also be advanced to the site of the lesion and engaged along the wall of the vessel to prevent restenosis from occurring within the vessel.

Retrieval of the embolic protection filter generally involves the use of a catheter or sheath having an inner lumen configured to collapse the filter and captured emboli therein. The ability of such retrieval devices to effectively trap the filter and its contents may depend in part on the size of the filter and filter wire, and the amount of emboli collected. The profile of the catheter or sheath may also affect the ability of the retrieval device to be delivered through the body.

### Summary of the Invention

The present invention is defined in claim 1 and pertains to retrieval devices for embolic protection filters. A retrieval device in accordance with an exemplary embodiment of the present invention includes an elongated shaft member having a proximal section, a distal section, and an inner lumen therethrough. A braided member coupled to the distal section of the elongated shaft member may be configured to receive and encapsulate an embolic protection filter therein. The braided member may include a number of filaments configured to radially expand when axially compressed. An elastomeric jacket disposed about at least a portion of the braided member may be used to encase the filaments. Methods of forming and using such devices are also disclosed.

### Brief Description of the Drawings

Figure 1 is a perspective view of a retrieval device in accordance with an exemplary embodiment of the present invention;
Figure 2 is a fragmentary cross-sectional view of a distal portion of the retrieval device of Figure 1;
Figure 3 is a perspective view of a braided member used in the construction of the retrieval device of Figure 1;
Figure 4 is a plan view of an embolic protection filter disposed within a vessel distal a lesion and placed stent;
Figure 5 is another plan view of the vessel shown in Figure 4, wherein a retrieval device is shown advanced along the filter wire;
Figure 6 is another plan view of the vessel shown in Figure 4, wherein the retrieval device has been further advanced along the filter wire to collapse the filter;
Figure 7 is another plan view of the vessel shown in Figure 4, wherein the filter is partially collapsed within the retrieval device;
Figure 8 is another plan view of the vessel shown in Figure 4, wherein the filter is fully encapsulated within the retrieval device; and
Figure 9 is another plan view of the vessel shown in Figure 4, wherein the retrieval device and encapsulated filter are withdrawn proximal the lesion and stent.

### Detailed Description of the Invention

The following description should be read with reference to the drawings, in which like elements in different drawings are numbered in like fashion. The drawings, which are not necessarily to scale, depict selected embodiments and are not intended to limit the scope of the invention. Although examples of construction, dimensions, and materials are illustrated for the various elements, those skilled in the art will recognize that many of the examples provided have suitable alternatives that may be utilized.

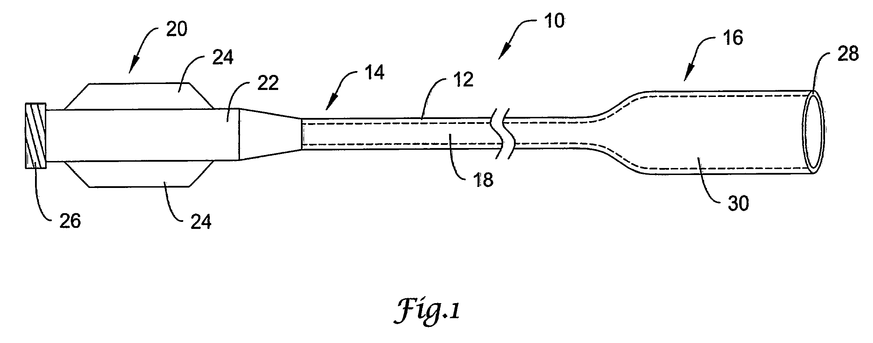

Figure 1 is a perspective view of a retrieval device 10 in accordance with an exemplary embodiment of the present invention. Retrieval device 10 includes an elongated shaft member 12 having a proximal section 14, a distal section 16, and an inner lumen 18 therethrough. The elongated shaft member 12 may include a suitably stiff material having sufficient column strength and rigidity to withstand buckling as the retrieval device 10 is advanced over an intravascular device such as an embolic protection filter. The wall thickness of the elongated shaft member 12 may be generally uniform along the length of the retrieval device 10, or may vary to alter the flexibility or bending characteristics of the device 10, as desired. Materials suitable for forming the elongated shaft member 12 include polymers such as polyether block amide (PEBA), or metal-polymer blends such as stainless steel reinforced hypotube. Polyether block amide (PEBA) is commercially available from Atochem Polymers of Birdsboro, Pennsylvania under the trade name PEBAX.

The proximal section 14 of the elongated shaft member 12 may be provided with a hub 20 having a handle 22 and a number of gripping fins 24 that allow the user to grip the proximal section 14 and manipulate the retrieval device 10 both axially and rotationally within the body. In certain embodiments, a vacuum source 26 coupled to the hub 20 and in fluid communication with the inner lumen 18 may be used to provide suction at the distal end 28 of the retrieval device 10. In use, the vacuum source 26 may be used to aspirate the embolic protection filter and any loose emboli into the retrieval device 10. Such suction force can be used in lieu of, or in addition to, manually manipulating the retrieval device 10 within the body to retrieve the intravascular device.

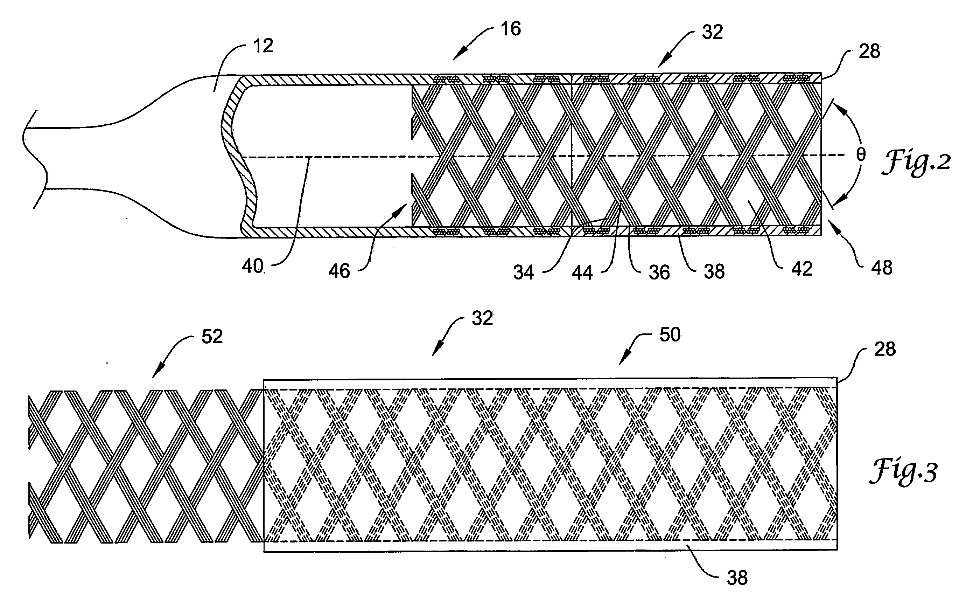

The distal section 16 of the elongated shaft member 12 may be flared slightly, forming a retrieval lumen 30 configured to expand and encapsulate the intravascular device therein. A braided member 32 (Figure 2) coupled to or formed integrally with the distal section 16 may be utilized to impart flexibility to the distal portion of the retrieval device 10 while maintaining the axial stiffness and rigidity characteristics generally exhibited by the remainder of the elongated shaft member 12.

Figure 2 is a fragmentary cross-sectional view of a distal portion of the retrieval device 10 of Figure 1. As shown in Figure 2, braided member 32 may include a number of filaments 34,36 encased within an elastomeric jacket 38 that can be configured to lie adjacent and flush with the distal section 16 of the elongated shaft member 12. The filaments 34,36 may be arranged generally in two sets of parallel helices wound in opposite directions about a common longitudinal axis 40, thus forming an inner lumen 42 of the braided member 32. The filaments 34,36 may intersect each other in an overlapping pattern at a number of interstices 44. The interstices 44 are configured to permit the two sets of filaments 34,36 to move with respect to each other, allowing the braided member 32 to radially expand and axially shorten when subjected to a compressive force, and conversely, radially shrink and axially elongate when subjected to a tensile force. The braid angle θ (*i.e*. the angle between two filaments in the longitudinal or axial direction) may be varied to alter the amount of radial expansion and axial elongation of the braided member 32, as desired. The ends 46,48 of each of the two sets of filaments 34,36 may be constrained to prevent the filaments 34,36 from fraying or unraveling.

The shape, thickness, or other characteristics of the braided member 32 may also vary to alter the characteristics of the retrieval device 10. In the exemplary embodiment depicted in Figure 1, the filaments 34,36 forming the braided member 32 are each made from multi-filar threads woven together to form filaments having a generally round shape. Other filament configurations may be employed, however, such as round wire, flat ribbon, threads, fibers, mono-filament, multi-filament, or combinations thereof. The thickness of the filaments 34,36 may vary in dimension to impart a greater or lesser amount of resistance to radial expansion to the braided member 32 as the intravascular device is loaded into the retrieval device 10. In general, the larger the size of filaments employed, the greater the resistance to radial expansion that results.

The expandability of the retrieval device 10 may also be due in part to the material composition of the braided member 32. The braided member 32 may comprise any number of suitable materials, including polymers, metals, metal alloys, metal-polymer composites, or metal-metal composites. Elastomeric materials may also be employed to impart a desired amount of flexibility to the braided member 32. Examples of suitable polymeric materials include, but are not limited to, polyethylene terapthalate (PET), polytetraflouroethylene (PTFE), polyurethane (Nylon) fluorinated ethylene propylene (FEP), ethylene tetrafluoroethylene (ETFE), polyurethane, polypropylene (PP), polyvinylchloride (PVC), polyether-ester, polyester, polyamide, elastomeric polyamides, block polyamide/ethers, polyether block amide (PEBA), silicones, polyethylene (PE), polyether-ether ketone (PEEK), polyimide (PI), polyetherimide (PEI), polyphenylene sulfide (PPS), polyphenylene oxide (PPO), polysulfone, perfluoro(propyl vinyl ether) (PFA), or other suitable materials, mixtures, combinations or copolymers thereof. Examples of suitable metals or metal alloys may include stainless steel, platinum, tungsten alloy and nickel-titanium alloy.

In certain embodiments, the filaments 34,36 may each be formed from a composite material configured to impart a desired characteristic to the braided member 32. For example, one or more stainless steel and nickel-titanium alloy wires can be wound together to form filaments having a desired characteristic such as superelasticity. Alternatively, in those embodiments employing wire or flat ribbon, for example, a composite material formed by a drawing, cladding or other suitable process may used to form filaments having a desired characteristic.

The filaments 34,36 may include a radiopaque metal such as gold, palladium, platinum, tantalum, and tungsten alloy, or may include a polymeric material loaded with a radiopaque agent such as barium sulfate (BaSO₄) or bismuth subcarbonate ((BiO)₂CO₃). Radiopaque materials are understood to be materials capable of producing a relatively bright image on a fluoroscopic monitor or other imaging device. When a radiopaque die is injected into the vessel at issue, the relatively bright image produced on the monitor can be used to determine the location of the retrieval device within the body.

Figure 3 is a perspective view of the braided member 32 used in the construction of the retrieval device 10 of Figure 1. As can be seen in Figure 3, an elastomeric jacket 38 may be placed about a distal section 50 of the braided member 32. The proximal section 52 of the braided member 32 may, in turn, be exposed to facilitate bonding of braided member 32 to the distal section 16 of the elongated shaft member 12.

In use, the elastomeric jacket 38 constrains and compresses the filaments 34,36 radially such that the braided member 32 assumes a generally low profile for delivery through the body. In addition, the elastomeric jacket 38 protects against the intrusion of other objects between the interstices 44 of the filaments 34,36. In some embodiments, the elastomeric jacket 38 may be loaded with a radiopaque additive (*e*.*g*. barium sulfate or bismuth subcarbonate) to further enhance the visibility of the braided member 32 under a fluoroscope.

The elastomeric jacket 38 may be formed of silicon, C-flex, urethane or other suitable material having sufficient elasticity to permit the braided member 32 to radially expand while maintaining axial stiffness and rigidity. To form the elastomeric jacket 38, an uncured elastomeric material may be placed in either liquid or thixotropic form over the distal section 50 of the braided member 32. The braided member 32 is then placed in a mold while the elastomeric material is allowed to cure and assume its final solid form. In another exemplary embodiment, the elastomeric jacket 38 may be formed by an extrusion process wherein the elastomeric material is extruded or poured over the distal section 50 of the braided member 32, and then allowed to cool to its final solid form. In yet another alternative embodiment, an injection-mold process such as insert molding may be employed to bond the elastomeric jacket 38 to the braided member 32.

The braided member 32 may be formed from a separate member that is attached to the distal section 16 of the elongated shaft member 12, or may be formed integral with the elongated shaft member 12. The braided member 32 can be attached to the elongated shaft member 12 using any number of suitable bonding techniques such as adhesion, laser welding, rf welding, soldering, or crimping. In one exemplary bonding technique, a thin piece of heat-shrinking tube can be placed about the distal section 16 of elongated shaft member 12 and the braided member 32, and then heated until the two members 12,32 meld together. The heat-shrink tube can then be removed from the retrieval device 10.

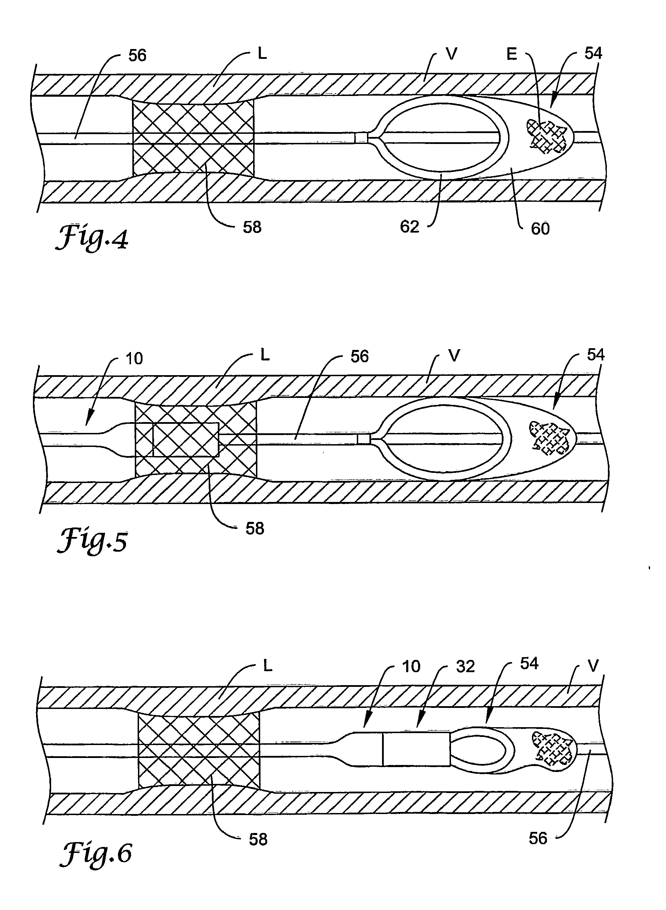

Referring now to Figures 4-9, an exemplary method of retrieving an intravascular device in accordance with the present invention will now be discussed with respect to retrieval device 10 described herein. In a first position depicted in Figure 4, an embolic protection filter 54 attached to a filter wire 56 is shown positioned within a vessel V distal a lesion L. A previously placed stent 58 is also shown advanced along the filter wire 56 and positioned across the site of the lesion L to prevent restenosis from occurring subsequent to a therapeutic procedure such as an angioplasty or atherectomy. As can be seen in Figure 4, the embolic protection filter 54 may include a filter mesh or membrane 60 operatively coupled to a proximal support hoop 62, which is biased to expand the filter mesh or membrane 60 within the vessel V. Throughout the course of treatment, embolic debris E dislodged from the vessel wall enters the embolic protection filter 54 through the proximal support hoop 62, where it is collected and stored within the filter mesh or membrane 60.

At the conclusion of the therapeutic procedure, the retrieval device 10 can be advanced along the filter wire 56 across the site of the lesion L, as shown in Figure 5. During delivery, the retrieval device 10 assumes a relatively low profile, allowing the device to be advanced through the stent 58 with minimal interference.

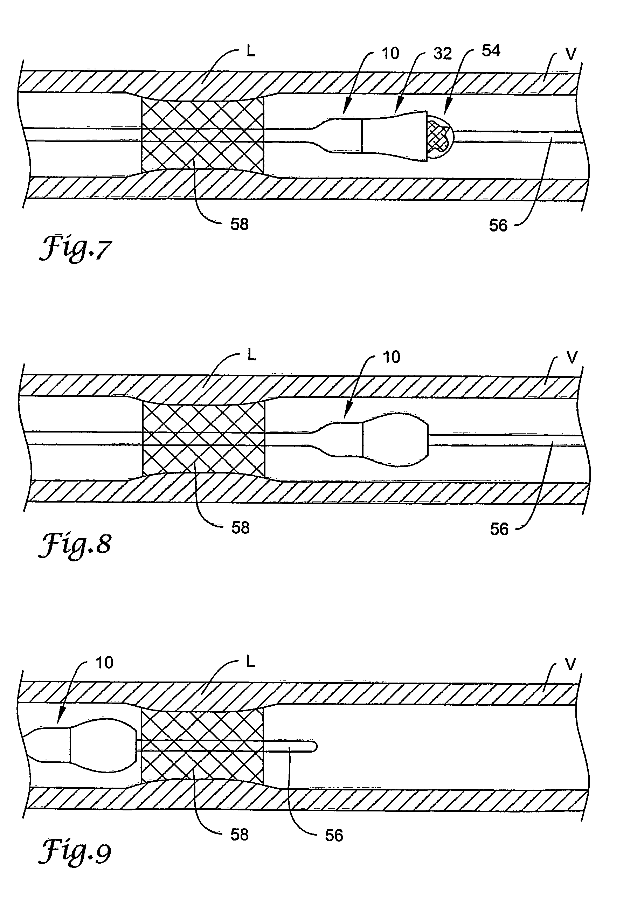

Continued advancement of the retrieval device 10 over the filter wire 56 causes the embolic protection filter 54 to begin to collapse within the braided member 32, as shown in Figure 6. As the embolic protection filter 54 is further forced therein, the braided member 32 compresses axially and expands radially, as shown in Figure 7. A suction force may also be applied to the distal end 28 of the retrieval device 10 to aspirate the embolic protection filter 54 and its contents therein. The embolic protection filter 54 and captured emboli E can then be completely encapsulated within the retrieval device 10, as shown in Figure 8. Subsequently, the retrieval device 10, embolic protection filter 54, and captured emboli E can then be removed from the vessel V along with the filter wire 56, as shown in Figure 9.

While Figures 4-9 illustrate the removal of an embolic protection filter from the body, it is contemplated that any number of other intravascular devices may be retrieved and/or delivered with the present invention. Examples of other intravascular devices may include stents, clot pullers, vena cava filters, atheterectomy devices, angioplasty devices, or the like.

Having thus described the several embodiments of the present invention, those of skill in the art will readily appreciate that other embodiments may be made and used which fall within the scope of the claims attached hereto. Numerous advantages of the invention covered by this document have been set forth in the foregoing description. It will be understood that this disclosure is, in many respects, only illustrative. Changes may be made in details, particularly in matters of shape, size and arrangement of parts without exceeding the scope of the invention as described in the appended claims.

## Claims

1. A medical device for retrieving an intravascular device from a body lumen, **caracterised by**:
an elongated shaft member (12) having a proximal section (14) and a flared distal section (16);
a braided member (32) having a proximal portion (52) and a distal portion (50), the proximal portion (52) of the braided member (32) coupled to the distal section (16) of the elongated shaft member (12) and the distal portion (50) of the braided member (32) extending distally of the distal section (16) of the elongate shaft member (12), the braided member (32) including a plurality of filaments (34, 36) and defining a radially expandable inner lumen configured to receive and encapsulate the intravascular device therein;
wherein said plurality of filaments (34, 36) are configured to radially expand when axially compressed.

2. The medical device of claim 1, further comprising an elastomeric jacket (38) encasing at least part of said braided member (32).

3. The medical device of claim 1, wherein the elongated shaft member (12) includes polyether block amide.

4. The medical device of claim 1, wherein said plurality of filaments (34, 36) are arranged in two sets of parallel helices wound in opposite directions about a common longitudinal axis.

5. The medical device of claim 1, wherein said plurality of filaments comprise multi-filar threads.

6. The medical device of claim 1, wherein said plurality of filaments comprise a composite material

7. The medical device of claim 6, wherein said composite material includes a radiopaque material.

8. The medical device of claim 1, wherein said plurality of filaments comprise a polymeric material.

9. The medical device of claim 1, wherein said plurality of filaments comprise a metal.

10. The medical device of claim 2, wherein said elastomeric jacket (38) includes silicone.

11. The medical device of claim 2, wherein said elastomeric jacket (38) includes urethane.

12. The medical device of claim 2, wherein .said elastomeric jacket (38) includes C-flex.

13. The medical device of claim 2, wherein said elastomeric jacket (38) includes a radiopaque material.

## Patentansprüche

1. Medizinische Vorrichtung zur Rückholung einer intravaskulären Vorrichtung aus einem Körperlumen, **gekennzeichnet durch**:
ein langgestrecktes Schaftteil (12) mit einem proximalen Abschnitt (14) und einem aufgeweiteten distalen Abschnitt (16);
ein geflochtenes Element (32) mit einem proximalen Teil (52) und einem distalen Teil (50), wobei der proximale Teil (52) des geflochtenen Elements (32) mit dem distalen Abschnitt (16) des langgestreckten Schaftteils (12) gekoppelt ist und der distale Teil (50) des geflochtenen Elements (32) sich distal zum distalen Abschnitt (16) des langgestreckten Schaftteils (12) erstreckt, wobei das geflochtene Element (32) mehrere Filamente (34, 36) aufweist und ein radial expandierbares Innenlumen definiert, das dafür konfiguriert ist, die intravaskuläre Vorrichtung darin aufzunehmen und einzukapseln;
wobei die mehreren Filamente (34, 36) dafür konfiguriert sind, bei axialem Druck radial zu expandieren.

2. Medizinische Vorrichtung nach Anspruch 1, ferner mit einem Elastomermantel (38), der zumindest einen Teil des geflochtenen Elements (32) umhüllt.

3. Medizinische Vorrichtung nach Anspruch 1, wobei das langgestreckte Schaftteil (12) Polyether-Block-Amid aufweist.

4. Medizinische Vorrichtung nach Anspruch 1, wobei die mehreren Filamente (34, 36) in zwei Sätzen von parallelen Schraubenlinien angeordnet sind, die in entgegengesetzten Richtungen um eine gemeinsame Längsachse gewickelt sind.

5. Medizinische Vorrichtung nach Anspruch 1, wobei die mehreren Filamente Multifilfäden aufweisen.

6. Medizinische Vorrichtung nach Anspruch 1, wobei die mehreren Filamente ein Verbundmaterial aufweisen.

7. Medizinische Vorrichtung nach Anspruch 6, wobei das Verbundmaterial ein strahlenundurchlässiges Material aufweist.

8. Medizinische Vorrichtung nach Anspruch 1, wobei die mehreren Filamente ein Polymermaterial aufweisen.

9. Medizinische Vorrichtung nach Anspruch 1, wobei die mehreren Filamente ein Metall aufweisen.

10. Medizinische Vorrichtung nach Anspruch 2, wobei der Elastomermantel (38) Silikon aufweist.

11. Medizinische Vorrichtung nach Anspruch 2, wobei der Elastomermantel (38) Urethan aufweist.

12. Medizinische Vorrichtung nach Anspruch 2, wobei der Elastomermantel (38) C-Flex aufweist.

13. Medizinische Vorrichtung nach Anspruch 2, wobei der Elastomermantel (38) ein strahlenundurchlässiges Material aufweist.

## Revendications

1. Dispositif médical pour extraire un dispositif intra-vasculaire d'une lumière corporelle, **caractérisé par** :
un élément d'arbre allongé (12) ayant une section proximale (14) et une section distale évasée (16) ;
un élément ramifié (32) ayant une partie proximale (52) et une partie distale (50), la partie proximale (52) de l'élément ramifié (32) étant couplée à la section distale (16) de l'élément d'arbre allongé (12) et la partie distale (50) de l'élément ramifié (32) s'étendant de manière distale depuis la section distale (16) de l'élément d'arbre allongé (12), l'élément ramifié (32) comprenant une pluralité de filaments (34, 36) et définissant une lumière interne extensible radialement configurée pour recevoir et encapsuler le dispositif intra-vasculaire à l'intérieur ;
dans lequel ladite pluralité de filaments (34, 36) est configurée de façon à s'étendre radialement lorsqu'elle est axialement comprimée.

2. Dispositif médical selon la revendication 1, comprenant en outre un chemisage en élastomère (38) englobant au moins une partie dudit élément ramifié (32).

3. Dispositif médical selon la revendication 1, dans lequel l'élément d'arbre allongé (12) comprend un amide en bloc de polyéther.

4. Dispositif médical selon la revendication 1, dans lequel ladite pluralité de filaments (34, 36) est disposée en deux ensembles d'hélices parallèles enroulées dans des directions opposées autour d'un axe longitudinal commun.

5. Dispositif médical selon la revendication 1, dans lequel ladite pluralité de filaments comprend des filets multifilaires.

6. Dispositif médical selon la revendication 1, dans lequel ladite pluralité de filaments comprend un matériau composite.

7. Dispositif médical selon la revendication 6, dans lequel ledit matériau composite comprend un matériau radio-opaque.

8. Dispositif médical selon la revendication 1, dans lequel ladite pluralité de filaments comprend un matériau polymère.

9. Dispositif médical selon la revendication 1, dans lequel ladite pluralité de filaments comprend un métal.

10. Dispositif médical selon la revendication 2, dans lequel ledit chemisage en élastomère (38) comprend du silicone.

11. Dispositif médical selon la revendication 2, dans lequel ledit chemisage en élastomère (38) comprend de l'uréthane.

12. Dispositif médical selon la revendication 2, dans lequel ledit chemisage en élastomère (38) comprend du C-flex.

13. Dispositif médical selon la revendication 2, dans lequel ledit chemisage en élastomère (38) comprend un matériau radio-opaque.
